(19) Europäisches Patentamt
European Patent Office
Office européen des brevets

(11) Publication number: **0 337 489 A2**

(12) **EUROPEAN PATENT APPLICATION**

(21) Application number: **89106741.5**

(22) Date of filing: **14.04.89**

(51) Int. Cl.4: **C07C 79/46 , C09K 19/20 , C09K 19/30 , C09K 19/24 , C07C 69/75 , C07C 107/06 , C07C 105/00 , C07C 121/75**

(30) Priority: **15.04.88 PL 271848**

(43) Date of publication of application: **18.10.89 Bulletin 89/42**

(84) Designated Contracting States: **DE FR GB**

(71) Applicant: **UNIWERSYTET WARSZAWSKI**
**ul. Krakowskie Przedmiescie 26/28**
**PL-00-927 Warszawa(PL)**

(72) Inventor: **Pyzuk, Wieslaw Jan**
**ul. Nowolipki 10 m. 44**
**PL-00-151 Warszawa(PL)**
Inventor: **Boncza-Tomaszewski, Zbigniew**
**ul. Niemirowska 1 m. 57**
**PL-02-921 Warszawa(PL)**
Inventor: **Krupkowski, Teodor**
**ul. Bonifacego 75 m. 114**
**PL-02-936 Warszawa(PL)**

(74) Representative: **von Füner, Alexander, Dr. et al**
**Patentanwälte v. Füner, Ebbinghaus, Finck**
**Mariahilfplatz 2 & 3**
**D-8000 München 90(DE)**

(54) **New derivatives of phenyl 3-phenylbutyrate, a method of obtaining new derivatives of phenyl 3-phenylbutyrate and the application of new derivatives of phenyl 3-phenylbutyrate for liquid crystalline mixtures.**

(57) The subject of the invention are new derivatives of phenyl 3-phenylbutyrate and a method of obtaining new derivatives of phenyl 3-phenylbutyrate of the general formula 1,

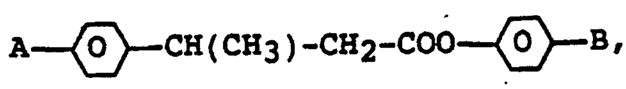

wherein
A denotes a hydrogen atom, the radical $C_1$-$C_{12}$-alkyl, the residue -O($C_1$-$C_{12}$-alkyl), -CO($C_1$-$C_{12}$-alkyl), -COO($C_1$-$C_{12}$-alkyl), -OOC($C_1$-$C_{12}$-alkyl) and the group -CN, -NCS or $-NO_2$,
B denotes residues of formulas

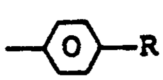 (1 a), 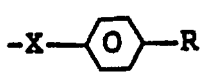 (1 b) or 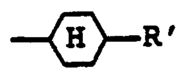 (1 c),

wherein R denotes substituents mentioned for A, R′ denotes the radical $C_1$-$C_{12}$-alkyl, while X denotes the group -N=N-, -N(O)N-, -COO- or -OOC-,
consisting in that the acid of the general formula 2

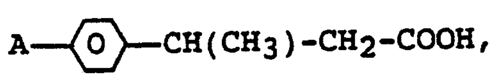

EP 0 337 489 A2

wherein A has the above mentioned meaning,
or in the form of an acid chloride, is reacted with phenol of the general formula 3

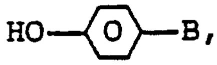

wherein B has the above mentioned meaning.

New derivatives of phenyl 3-phenylbutyrate are applied for the preparation of chiral or achiral liquid-crystalline mixtures.

# NEW DERIVATIVES OF PHENYL 3-PHENYLBUTYRATE, A METHOD OF OBTAINING NEW DERIVATIVES OF PHENYL 3-PHENYLBUTYRATE AND THE APPLICATION OF NEW DERIVATIVES OF PHENYL 3-PHENYL-BUTYRATE FOR LIQUID CRYSTALLINE MIXTURES

A subject of the invention are new derivatives of phenyl 3-phenylbutyrate of the general formula 1

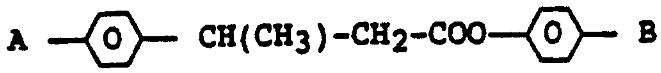

wherein
A denotes a hydrogen atom, the radical $C_1$-$C_{12}$-alkyl, the residue -O($C_1$-$C_{12}$-alkyl), -CO($C_1$-$C_{12}$-alkyl), -COO($C_1$-$C_{12}$-alkyl), -OOC($C_1$-$C_{12}$-alkyl) and the group -CN, -NCS or -$NO_2$,
B denotes residues of formulas

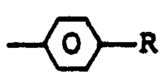 (1 a), 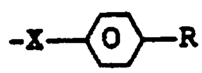 (1 b) or 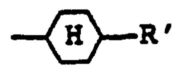 (1 c),

wherein
R denotes substituents mentioned for A, R′ denotes the radical $C_1$-$C_{12}$-alkyl, while X denotes the group -N=N-, -N(O)N-, -COO- or -OOC.

A further subject of the invention is also the method of obtaining new derivatives of phenyl 3-phenylbutyrate of the general formula 1

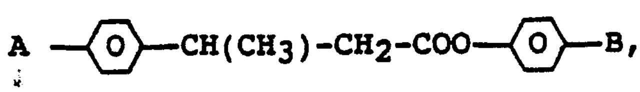

wherein A and B have the above mentioned meaning,
as well as the application of new derivatives of phenyl 3-phenylbutyrate for the preparation of chiral or racemic liquid crystalline mixtures.

There are known individual derivatives of phenyl 4-phenylbutyrate forming monotropic nematic phases, for example, such as the compound 9671 according to Demus, Zaschke: "Flüssige Kirstalle in Tabellen II", Leipzig, 1984. The said compounds, since they are devoid of an asymmetric carbon atom, do not form chiral liquid-crystalline phases. There is also known a derivative of phenyl 2-phenylbutyrate forming a monotropic smectic A phase (compound 9065 according to Demus, Zaschke: op. cit.). There are also known azo derivatives of phenyl 2-phenyl-iso-butyrate forming monotropic and enantiotropic nematic and chiral phases (compounds 2340, 2341, 2356c and 2356d according to Demus, Demus, Zaschke: "Flüssige Kristalle in Tabellen", Leipzig, 1974), while derivatives of phenyl 3-phenylbutyrate of liquid-crystalline properties have not been described so far in the literature.

It has been unexpectedly found out that enantiotropic or monotropic liquid-crystalline phases occur in derivatives of phenyl 3-phenylbutyrate, containing in a molecule three six-membered rings, two of which are connected with each other directly or through an ester, an azo- or an azoxy-group.

The method of obtaining new derivatives of phenyl 3-phenylbutyrate of the general formula 1

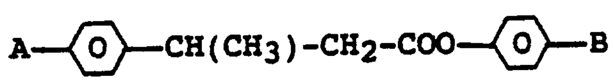

wherein
A denotes a hydrogen atom, the radical $C_1$-$C_{12}$-alkyl, the residue -O($C_1$-$C_{12}$-alkyl), -CO($C_1$-$C_{12}$-alkyl), -COO($C_1$-$C_{12}$-alkyl), -OOC($C_1$-$C_{12}$-alkyl) and the group -CN, -NCS or -$NO_2$,
B denotes residues of the formulas

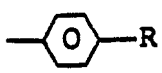 (1 a), 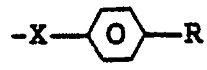 (1 b) or 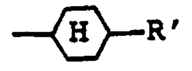 (1 c),

wherein R denotes substituents mentioned for A, R′ denotes the radical $C_1$-$C_{12}$-alkyl, while X denotes the group -N=N, -N(O)N, -COO-, or -OOC-,
according to the invention consists in that the acid of the general formula 2

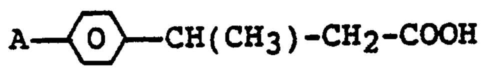,

wherein A has the above mentioned meaning,
or an acid chloride is reacted with phenol of the general formula 3

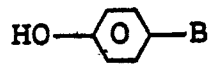,

wherein B has the above mentioned meaning.

-COO($C_1$-$C_{12}$-alkyl) means that this carboxylic group is bonded to the alkyl group by the oxygen atom and -OOC($C_1$-$C_{12}$-alkyl) means that the carboxylic group is bonded to the alkyl group by the carbon atom.

The same refers to X = -COO or -OOC. It is intended to express that X is bonded to the ring by the carbon atom or the oxygen atom, respectively.

In the method according to the invention the acid of the general formula 2

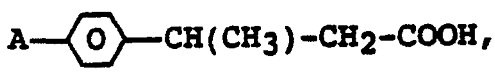,

wherein A has the above mentioned meaning,
or the chloride of said acid is applied in the form of a pure optical isomer, mixtures of enantiomers with prevailance of one of them, or in the form of a racemate, owing to which chiral or racemic esters of the general formula 1

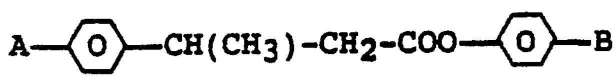,

wherein A and B have the above mentioned meaning,
are obtained. Chiral acids of the general formula 2

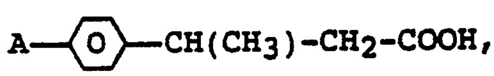,

wherein A has the above mentioned meaning,
are obtained by a resolution, i.e. division or partition of racemates or by a synthesis from chiral 3-phenylbutyric acids.

In the method according to the invention, as the acid of general formula 2

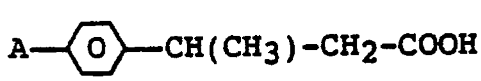,

wherein A has the above mentioned meaning,
also curcumic acid being the product of natural raw materials processing is applied.

In the method according to the invention as phenol of the general formula 3

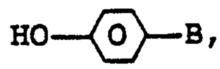

in the case when B denotes the residue of the formula 1 c

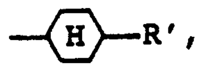

wherein R′ has the above mentioned meaning, a substituted isomer in the trans configuration is applied.

Esterification reactions between the acid of the formula 2

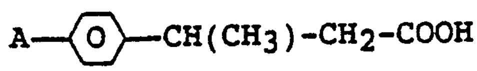

and phenol of the formula 3

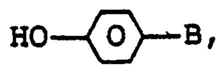

in which A and B have the above mentioned meaning, are conducted in the inert solvent medium in the presence of known reaction catalysts and condensing agents. In the preferable case of esterification of the acid in the form of acid chloride the reaction is conducted in the presence of a hydrogen chloride bonding agent or in the medium of a solvent being at the same time a hydrogen chloride bonding agent.

As the solvent one can apply any solvent which does not disturb considerably the reaction of this type. It is possible, for example, to apply individual aromatic hydrocarbons or in mixtures. As the hydrogen chloride bonding agent one can apply aromatic heterocyclic nitrogen bases, for example, such as pyridine, quinoline, isoquinoline or tertiary aliphatic amines. The reaction can be conducted at ambient temperature or at an elevated temperature, for example, from 50°C to the boiling point of the solution, with the application of reactants in equimolar amounts, or preferably with the application of a double or triple excess of the acid or its acid chloride. The reaction product is isolated by the known methods.

New derivatives of phenyl 3-phenylbutyrate of the general formula 1

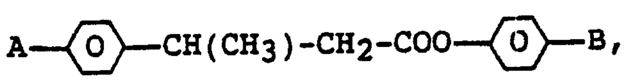

wherein A and B have the above mentioned meanings, obtained by the method according to the invention, are monotropic or enantiotropic liquid crystals, depending on the selection of substituents. For example, an enantiotropic nematic is the compound with substituents A = $-OCH_3$, X = -N=N-, R = $-OC_2H_5$, while the monotropic nematic phase is easily observed in the case when strongly polar groups as $-NO_2$ and -CN are present in the molecule.

Owing to its properties, the new derivatives of phenyl 3-phenylbutyrate are excellently applicable as components of liquid-crystalline mixtures used in the information displays, especially those based on chiral nematics or smectics. The compounds, which are obtained by the method being the subject of the invention, are characterized by particularly advantageous properties, having in the chiral form bigger optical activity and twisting power in comparison with derivatives of 2-methylbutanol (Demus, Zaschke: op. cit.) or N-1-phenyl-ethyl-amide (Polish provisional Patent No. 126 318). Compounds containing the azo group can be used also as liquid-crystalline dyes.

The subject of the invention is illustrated by the following examples, which, however, do not limit its scope. The tabel contains physical properties of a few new derivatives of phenyl 3-phenylbutyrate obtained by the method according to the invention.

Example 1: dl-3-phenylbutyrate-(4′-methoxy-phenylazo)phenyl-4

4.24 g (0.2 mmole) of 4-hydroxy-4′-methoxyazobenzene was dissolved in 25 $cm^3$ of anhydrous pyridine and 7.3 g (0.4 mmole) of dl-3-phenylbutyric acid chloride was added. The mixture was left at room temperature for 100 hours, and then, it was poured on water with ice. The reaction product was extracted with methylene chloride (3 x 100 $cm^3$). The extract was washed, in sequence, with diluted solutions of potassium carbonate, hydrochloric acid and water, dried with anhydrous magnesium sulphate and the solvent was distilled off. After purification of the crystalline residue by the column chromatography method (silica gel, benzene eluent) 6.88 g (92% yield) of the product in the form of light orange crystals was obtained. Physical properties of the title compound, specified in the table, defined after crystallization from methanol, do not change after a few-weeks' exposure of the compound of sunlight.

Example 2: l-3-phenylbutyrate-(4′-methoxyphenylazo)phenyl-4

4.0g of l-3-phenylbutyric acid of high optical purity (ester with l-methol: m.p. 47-48 °C, specific rotation $[\alpha]_D^{20}$ -83.1° in the 1.0% benzene solution) was gently heated to boiling for 4 hours with 4.0 g of thionyl chloride which was next distilled off at atmospheric pressure. To crude chloride of l-3-phenylbutyric acid 2.3 g of 4- hydroxy-4′-methoxyazobenzene in 12 $cm^3$ of pyridine was added. Proceeding further like in Example 1, 3.78 g of the title ester (92% yield) of the properties presented in the table was obtained.

Example 3: dl-3-(4′-nitro)phenylbutyrate-(4′-n-propyl-transcyclohexyl)phenyl

dl-3-(4′-nitro) phenylbutyric acid of the melting point of 167-168 °C was heated to boiling with a twice as big by weight amount of thionyl chloride, the excess of which was next distilled off at atmospheric pressure. 1.0 g (4.7 mmole) of crude acid chloride thus obtained was dissolved in 10 $cm^3$ of anhydrous pyridine and 0.7 g (3.7 mmole) of 4-propyl-trans-cyclohexylphenol of the transition temperature from the smectic to the isotropic phase of 137-138 °C was added. The mixture was heated in the argon atmosphere under a reflux condenser for 1.5 hours, and then was poured out to water with ice. The reaction product was extracted, the extract was washed, dried and the solvent was distilled off like in Example 1. After purification of the crystalline residue by the column chromatography method (neutral aluminium oxide, benzene eluent), 1.1 g (81% yield) of the product was obtained in the form of pale yellowish crystals. Physical properties of the title compound, specified in the table, were defined after crystallization from methanol.

Table

| Example | Functional groups A | X | B R | R′ | Optical form | Phase transition temperatures ($^{o}C$) | $[\alpha]^{20}_{D}$ |
|---|---|---|---|---|---|---|---|
| 1 | -H | -N=N- | $-OCH_3$ | | dl- | K 98-98.5 (N 53.5) I | 0 |
| 2 | -H | -N=N- | $-OCH_3$ | | l- | K 86 (Ch 54) I | $-88.4^{o}$ |
| 3 | $-NO_2$ | | $-C_3H_7$ | | dl- | K 79.5-80 (N 27) I | 0 |
| 4 | $-CH_3$ | | $-COOC_5H_{11}$ | | dl- | K 66-67 (N 17) I | 0 |
| 5 | $-COC_{11}H_{23}$ | | -CN | | dl- | K 91-93 (N 72) I | 0 |
| 6 | $-NO_2$ | | -CN | | dl- | K 118-119 (N 40) I | 0 |
| 7 | $-NO_2$ | -N=N- | $-OCH_3$ | | d- | K 96-97 (Ch 86) I | $175^{o}$ * |

Explanations:

K = crystalline phase
I = isotropic phase
N = nematic phase
Ch = cholesteric phase

$[\alpha]^{20}_{D}$ - specific rotation measured in a 1.0% chloroform solution

----------

* $[\alpha]^{25}_{D}$

## Claims

1. New derivatives of phenyl 3-phenylbutyrate of the general formula 1

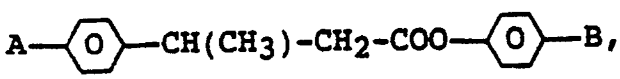

wherein
A denotes a hydrogen atom, the radical $C_1$-$C_{12}$-alkyl, the residue -O($C_1$-$C_{12}$-alkyl), -CO($C_1$-$C_{12}$-alkyl), -COO($C_1$-$C_{12}$-alkyl), -OOC($C_1$-$C_{12}$-alkyl) and the group -CN, -NCS or $-NO_2$,
B denotes residues of formulas

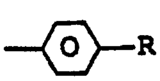 (1 a), 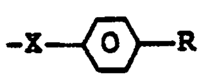 (1 b) or 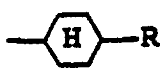 (1 c),

wherein R denotes substituents mentioned for A, R′ denotes the radical $C_1$-$C_{12}$-alkyl, while X denotes the group -N= N-, -N(O)N-, -COO- or -OOC-.

2. A method of obtaining new derivatives of phenyl 3-phenylbutyrate of the general formula 1,

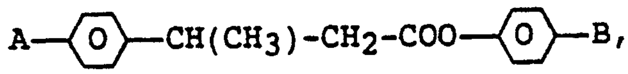

wherein
A denotes a hydrogen atom, the radical $C_1$-$C_{12}$-alkyl, the residue -O($C_1$-$C_{12}$-alkyl), -CO($C_1$-$C_{12}$-alkyl), -COO($C_1$-$C_{12}$-alkyl), -OOC($C_1$-$C_{12}$-alkyl) and the group -CN, -NCS or -$NO_2$,
B denotes residues of formulas

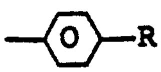 (1 a), 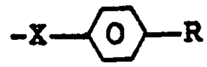 (1 b) or 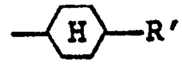 (1 c),

wherein R denotes substituents mentioned for A, R′ denotes the radical $C_1$-$C_{12}$-alkyl, while X denotes the group -N=N-, -N(O)N-, -COO- or -OOC-,
**characterized** in that the acid of the general formula 2

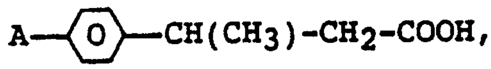

wherein A has the above mentioned meaning,
or in the form of an acid chloride, is reacted with phenol of the general formula 3

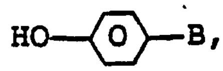

wherein B has the above mentioned meaning.

3. A method according to Claim 2, **characterized** in that the acid of the general formula 2

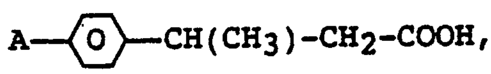

wherein A has the above mentioned meaning,
or a chloride of said acid is applied in the form of a pure optical isomer, a mixture of enantiomers with prevailance of one of them, or in the form of a racemate.

4. A method according to Claim 2 or 3, **characterized** in that as the acid of the general formula 2

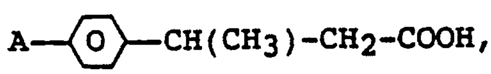

wherein A has the above mentioned meaning,
curcumic acid is applied.

5. A method according to Claim 2, **characterized** in that phenol of general formula 3

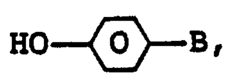

where B denotes the residue of formula 1 c

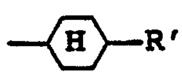

wherein R′ has the above mentioned meaning, is applied in the form of a substituted isomer in the trans configuration.

6. The application of new derivatives of phenyl 3-phenylbutyrate for the preparation of chiral or racemic liquid-crystalline mixtures.